# EUROPEAN PATENT SPECIFICATION

(11) **EP 4 408 128 B1**
(45) Date of publication and mention of the grant of the patent: **17.06.2026**
(21) Application number: 22872542.0
(22) Date of filing: 25.07.2022
(51) Int. Cl.: H05H 13/00, A61N 5/10, H05H 7/08

(54) **CIRCULAR ACCELERATOR AND PARTICLE BEAM RADIOTHERAPY SYSTEM**
KREISBESCHLEUNIGER UND TEILCHENSTRAHLSTRAHLENTHERAPIESYSTEM
ACCÉLÉRATEUR CIRCULAIRE ET SYSTÈME DE RADIOTHÉRAPIE À FAISCEAU DE PARTICULES

(30) Priority: 24.09.2021 JP 2021155862
(43) Date of publication of application: 31.07.2024
(73) Proprietor: Hitachi High-Tech Corporation, Minato-ku Tokyo 105-6409 (JP)
(72) Inventor: SEKI, Takayoshi, Tokyo 100-8280 (JP)
(74) Representative: MERH-IP Matias Erny Reichl Hoffmann Patentanwälte PartG mbB
(86) International application number: PCT/JP2022/028592
(87) International publication number: WO 2023/047786

(56) References cited:
- WO-A1-2020/166116
- JP-A- 2011 523 185
- JP-A- 2019 096 405
- JP-A- 2019 169 255
- JP-A- S5 574 050
- US-A1- 2011 068 717
- US-A1- 2017 186 581

## Description

### Technical Field

The present invention relates to a circular accelerator, and a particle therapy system.

### Background Art

As a background art of the present technical field, there is a technique described in PTL 1.

PTL 1 describes "includes a second internal ion source (2) for generating the same particle ions as the first internal ion source (1), and the cyclotron is further capable of generating an energy particle beam generated by either the first internal ion source or the second internal ion source or simultaneously by both ion sources" and describes "the central vertical axis is defined as an axis that passes through the center of the cyclotron and is parallel to the direction of the magnetic field inside the cyclotron. According to another embodiment, the ion sources are placed at substantially the same distance from the central axis, but not necessarily symmetrically with respect to the central axis". Furthermore, PTL 2 discloses an ion source, primarily for substrate deposition, including inner and outer magnetic poles that create a closed-loop path for electron acceleration, wherein a gas injection port is integrated directly into one of the magnetic poles. Thereby, the working gas can be supplied into the electron acceleration loop where plasma is formed. PTL 3 discloses an internal ion source for a circular accelerator and describes an Electron Cyclotron Resonance (ECR) type source, wherein microwaves from an external generator are guided via a waveguide into a hole in the yoke of the main pole. Within this hole, a secondary magnetic field generator creates a field opposing the main accelerator field. Eventually, PTL 4 discloses a cyclotron with two internal ion sources arranged within the central region, thereby allowing one source to act as a backup for the other to increase the uptime and reliability of the accelerator.

### Citation List

### Patent Literature

PTL 1: JP 2011-523185 A
PTL 2: US 2017 / 186 581 A1
PTL 3: WO 2020 / 166 116 A1
PTL 4: US 2011 / 068 717 A1

### Summary of Invention

### Technical Problem

PTL 1 describes that two ion sources are arranged in a circulating plane inside the cyclotron, and the two ion sources are switched to be used, thereby improving an operation rate and reliability.

However, the technique described in PTL 1 is not considered to be applied to the case of a circular accelerator having an acceleration electrode with one acceleration gap. Therefore, for example, there is a problem that the ion source interferes with the circulating beam and the extraction current from the accelerator decreases, or it is difficult to perform installment. In addition, it is necessary to adjust the installation positions of the respective ion sources so as not to interfere, so that the time required for exchanging the ion source becomes long, and the operation rate of the apparatus is lowered, which is problematic. Furthermore, it is difficult to install two or more ion sources, and further it is difficult to improve the operation rate.

The present invention provides a circular accelerator and a particle therapy system in which an operation rate and maintainability of an apparatus are improved as compared with the related art.

### Solution to Problem

The invention is set out in the appended set of claims. Preferable embodiments are defined in the dependent claims.

### Advantageous Effects of Invention

According to the present invention, it is possible to improve an operation rate and maintainability of an apparatus as compared with the related art. Problems, configurations, and effects other than those described above will become apparent from the description of the following embodiments.

### Brief Description of Drawings

[FIG. 1] FIG. 1 is a diagram illustrating an overall configuration of a particle therapy system using a circular accelerator of the present invention.
[FIG. 2] FIG. 2 is a diagram illustrating a side cross section of the circular accelerator illustrated in FIG. 1.
[FIG. 3] FIG. 3 is a diagram illustrating a cross section of the circular accelerator illustrated in FIG. 1.
[FIG. 4] FIG. 4 is a diagram schematically illustrating a side surface around an ion source in FIG. 2.
[FIG. 5] FIG. 5 is a diagram illustrating a schematic structure of the ion source of FIG. 4 as viewed from below.
[FIG. 6] FIG. 6 is a diagram illustrating an outline of a case where the ion source of FIG. 4 is moved downward by a positioning guide.
[FIG. 7] FIG. 7 is a diagram illustrating another embodiment of the ion source of the present invention, and is a diagram illustrating an outline of a case where the ion source is moved by a positioning pin.
[FIG. 8] FIG. 8 is a diagram illustrating still another embodiment of the ion source of the present invention, and is a diagram illustrating a structure in a case where a gas introduction pipe is integrated.
[FIG. 9] FIG. 9 is a diagram illustrating a structure in a case where the ion source of FIG. 8 is moved downward in a case where the gas introduction pipe is integrated.

### Description of Embodiments

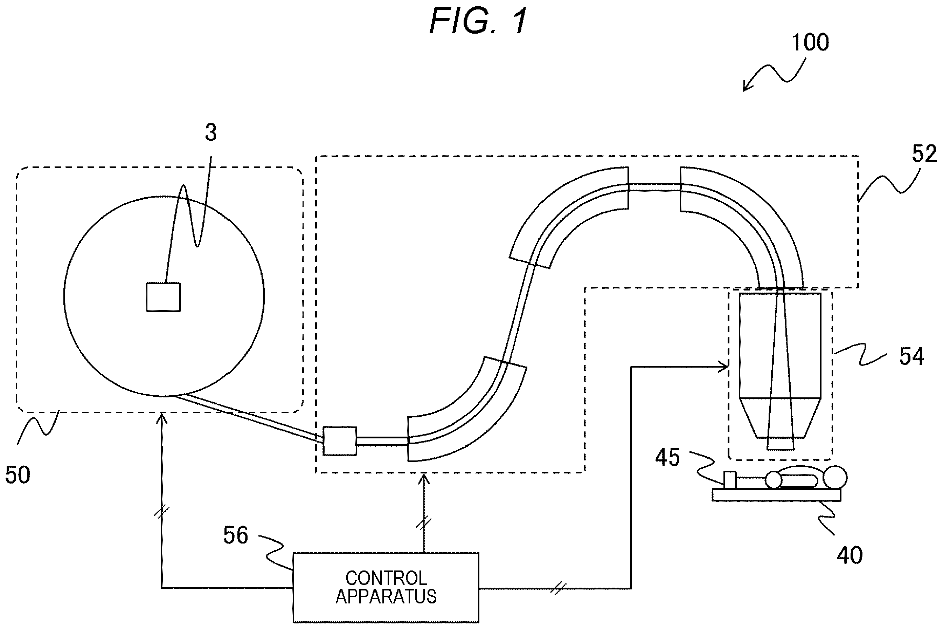

Embodiments of a circular accelerator and a particle therapy system according to the present invention will be described with reference to FIGS. 1 to 9. Note that in the drawings used in the present specification, the same or corresponding components are denoted by the same or similar reference numerals, and repeated description of these components may be omitted.

First, an overall configuration of the particle therapy system and a configuration of a related apparatus will be described with reference to FIG. 1. FIG. 1 is a diagram illustrating an overall configuration of the particle therapy system of the present embodiment.

In FIG. 1, a particle therapy system 100 includes a cyclotron type accelerator 50, a beam transport system 52, an irradiation apparatus 54, a treatment table 40, a control apparatus 56, and the like.

In the particle therapy system 100, ions generated by the ion source 3 are accelerated by the accelerator 50 to form an ion beam. The ion beam accelerated to desired energy is extracted from the accelerator 50 and transported to the irradiation apparatus 54 by the beam transport system 52. The transported ion beam is shaped to be conformal to target shape by the irradiation apparatus 54, and a predetermined amount of ion beam is irradiated to the target of a patient 45 lying on the treatment table 40. The operation of each apparatus and equipment, which includes the accelerator 50, in the particle therapy system 100 is controlled by the control apparatus 56.

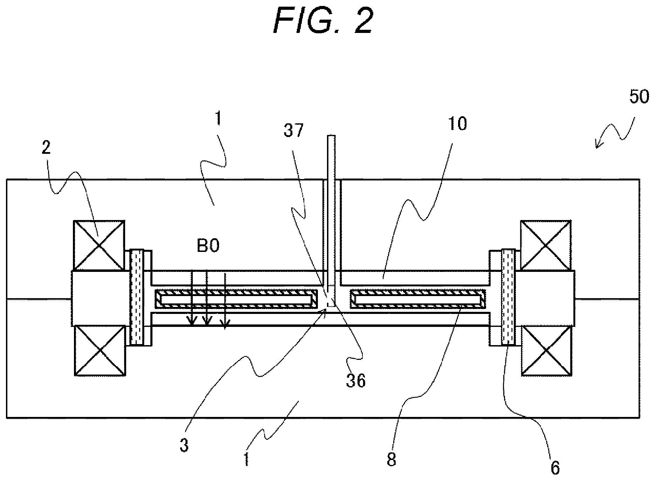

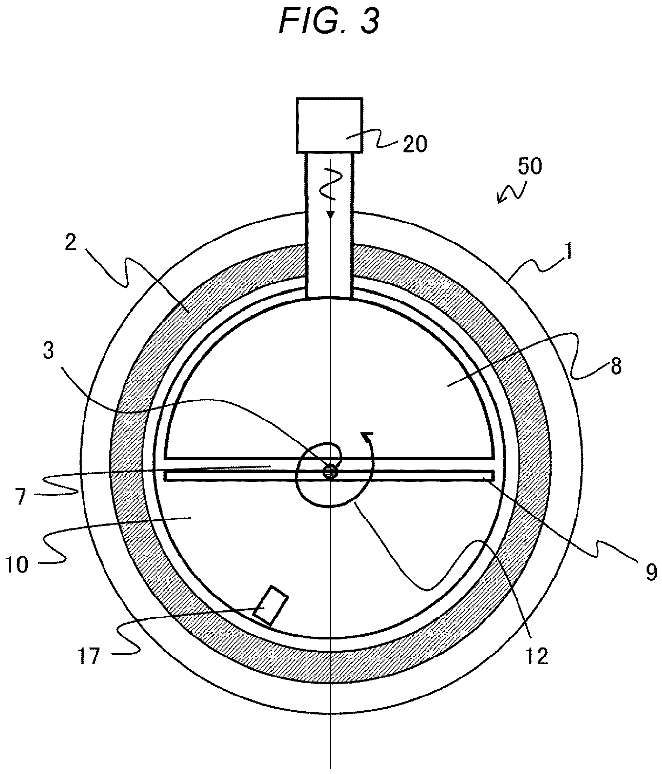

Next, the structure of the accelerator 50 will be described with reference to FIGS. 2 and 3. FIG. 2 is a cross-sectional view of a side surface of the accelerator of the present embodiment, and FIG. 3 is a transverse cross-sectional view.

As illustrated in FIGS. 2 and 3, the cyclotron type accelerator 50 includes a main magnetic pole 1, a circular coil 2, a vacuum chamber 6, a radiofrequency acceleration electrode 8, and the ion source 3.

The main magnetic pole 1 is a pair of magnetic bodies installed to oppose each other, and is made of, for example, iron. In the main magnetic pole 1, a pair of upper and lower magnetic poles 10 are provided to oppose each other so as to generate an orbit 12 of the beam, and a magnetic field is generated between the magnetic poles 10. A magnetic field B0 as illustrated in FIG. 2 is generated by the magnetic pole 10, and an inclined magnetic field is formed outward from the circulating center of the magnetic pole 10 to generate a focusing force of a circulating ion beam, thereby realizing a stable circulating. The surface shapes of the opposing surfaces, between a magnetic pole gap, of the pair of upper and lower magnetic poles 10 that generate the magnetic field B0 are symmetrical shapes. Alternatively, a magnetic pole shape is formed to be provided with unevenness in a traveling direction of the beam, thereby forming a magnetic pole having a shape of adding a convergence effect.

The vacuum chamber 6 is sandwiched by the main magnetic pole **1,** and one vacuum chamber is formed with the magnetic pole 10 as an inner surface, whereby configuring a magnetic circuit. The vacuum chamber 6 is a non-magnetic body. Note that a separate vacuum chamber in which the magnetic pole 10 does not serve as the inner surface of the vacuum chamber may be separately provided in the gap between the magnetic poles 10.

The circular coil 2 is installed closer to the atmosphere than the vacuum chamber 6, and generates a magnetic field of B0 between a pair of upper and lower main magnetic poles **1.** The circular coil 2 can generate a magnetic field similarly by a coil made of a normal conducting material or a coil made of a superconducting material. Note that the circular coil 2 may be installed in the vacuum chamber 6, and is not particularly limited.

The ion source 3 is arranged inside the magnetic pole 10 and passes through an extraction hole 37 from the plasma generated in a discharge chamber 36, and an extraction beam 15 is formed by the radiofrequency acceleration electrode 8 and a radiofrequency electric field generated between the ion source 3 and a ground electrode 9 having the same potential as the ion source 3 at a radio frequency supplied by a radiofrequency power supply 20. The extraction beam 15 formed circulates while tracing the helical orbit 12 by the magnetic field B0 generated by the magnetic pole 10 and the electric field generated in an acceleration gap 7 between the radiofrequency acceleration electrode 8 and the ground electrode 9, is accelerated every time of passing through the acceleration gap 7, is accelerated to predetermined energy while increasing energy, and is then extracted to the outside of the main magnetic pole 1.

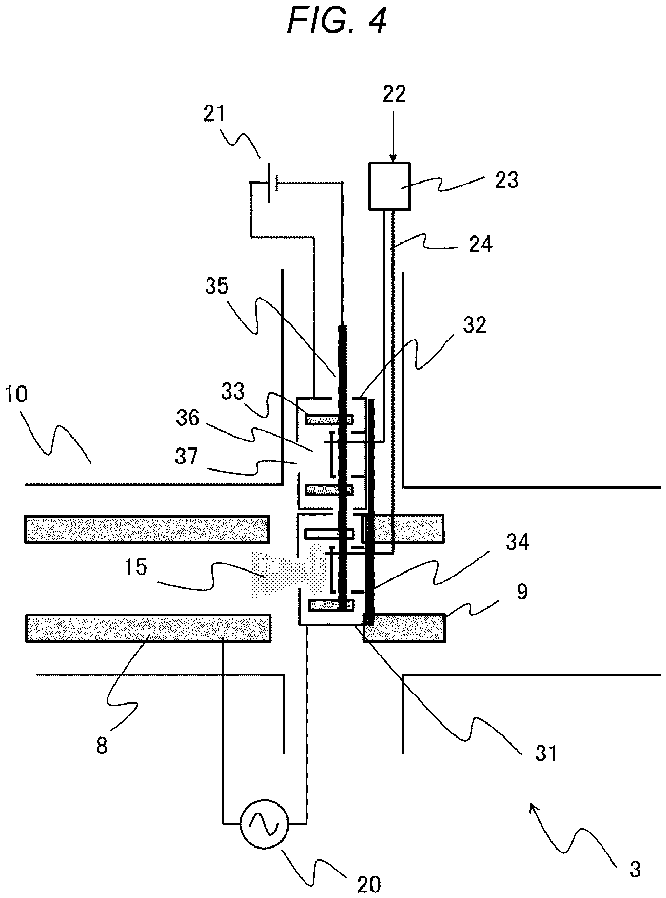

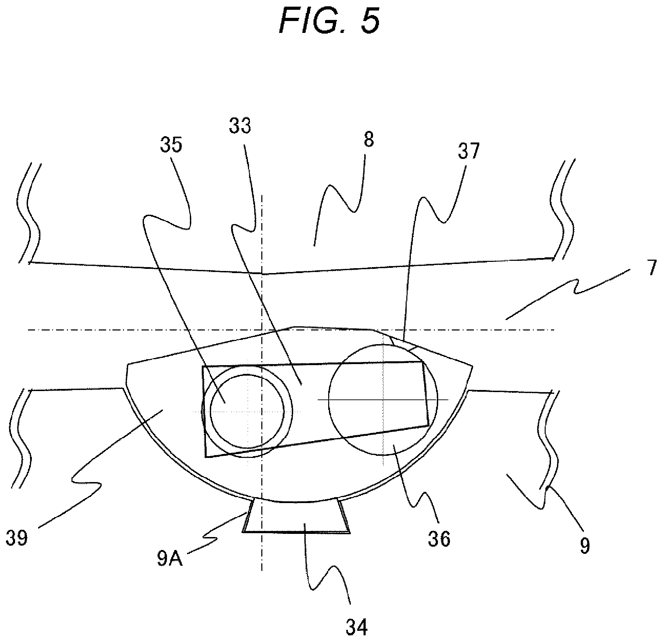

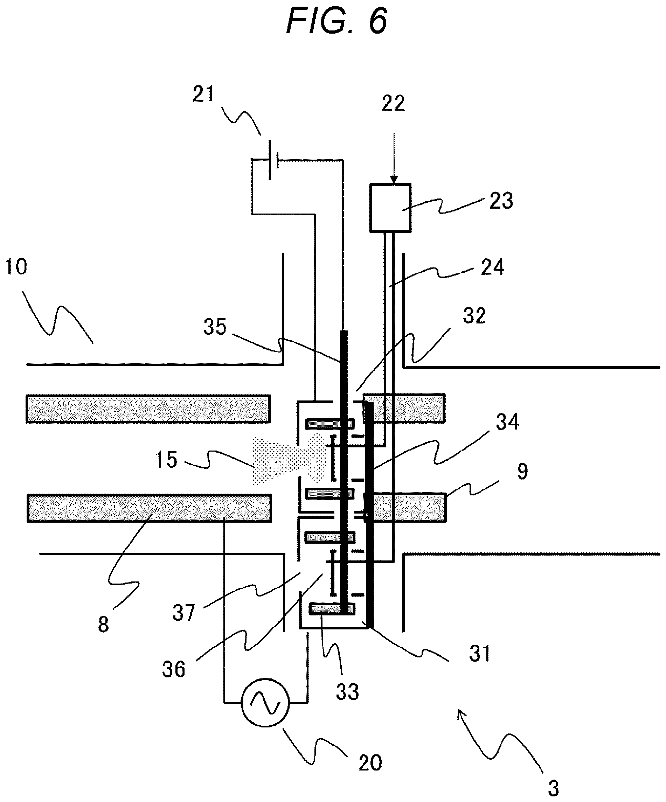

Next, details of the ion source 3 will be described with reference to FIGS. 4 to 6. FIG. 4 is a diagram illustrating details of the ion source 3 of FIG. 2, and is a diagram of a case where a first ion source 31 is operated in an example of a case where the first ion source 31 and a second ion source 32 are arranged in series in a direction parallel to the magnetic field generated by the magnetic pole 10. FIG. 5 is a diagram of the ion source 3 as viewed from below. FIG. 6 is a diagram of a case where the ion source 3 of FIG. 2 is moved downward and the second ion source 32 is operated.

The ion source 3 illustrated in FIG. 4 is installed between the opposing magnetic poles 10, and at least two discharge chambers 36 that generate ions in parallel with the central axis of the circulating ion beam are arranged linearly.

More specifically, the ion source 3 is configured such that two of the penning ionization gauge (PIG) type first ion source 31 and the second ion source 32 having the same configuration as the first ion source 31 are arranged side by side in a straight line parallel to the central axis of the circulating ion beam, that is, in an insertion direction of the ion source 3 into the main magnetic pole 1 (the same direction as the direction in which a plurality of the discharge chambers 36 is arranged), the first ion source including a cathode 33 that generates electrons, the discharge chamber 36 that generates ions, a discharge power supply 21, the extraction hole 37 for extracting ions from the discharge chamber 36, and a discharge vessel 39.

The ion source 3 is further provided with a gas pipe 24 for supplying a sample gas to each of the discharge chamber 36 of the first ion source 31 and the discharge chamber 36 of the second ion source 32.

Note that the number of the discharge chambers 36 of the ion source 3 is not limited to two, and a plurality of the discharge chambers can be arranged in an overlapping manner in a similar direction, and the number of the discharge chambers can be three or more.

In the ion source 3 illustrated in FIGS. 4 to 6, the cathode 33 of the first ion source 31 and the cathode 33 of the second ion source 32 are fixed to a common cathode supporting tool 35 as illustrated in FIGS. 4 and 5, and both the first ion source 31 and the second ion source 32 are arranged so as to sandwich the discharge chamber 36.

In the first ion source 31 and the second ion source 32, a voltage is fed between the cathode 33 and the discharge vessel 39 by the discharge power supply 21. When the cathode 33 is set to a negative potential with respect to the discharge vessel 39, electrons are extracted toward the discharge chamber 36.

The gas pipes 24 separately provided are connected to the discharge chamber 36 of the first ion source 31 and the discharge chamber 36 of the second ion source 32, respectively, and a sample gas 22 is supplied by a gas switcher 23 to the first ion source 31 positioned at the radiofrequency acceleration electrode 8. That is, the sample gas 22 is supplied only to the discharge chamber 36 to be operated in the ion source 3.

At this time, a voltage is fed to both cathodes 33 of the cathode 33 of the first ion source 31 and the cathode 33 of the second ion source 32, but in the second ion source 32 into which the sample gas 22 is not introduced, plasma is not generated, and the ion beam is not extracted. Since the plasma is not generated, the cathode 33 is not damaged by the ions of the plasma, and the lifetime is not affected.

In the ion source 3, the extraction hole 37 of the first ion source 31 and the extraction hole 37 of the second ion source 32 are arranged in a line in the insertion direction so as to be aligned in a straight line parallel to the central axis of the circulating ion beam.

In addition, a common positioning guide 34 is provided in the first ion source 31 and the second ion source 32, and the positioning guide 34 is slid and inserted to be aligned with a notch 9A provided in the ground electrode 9. As a result, the positions of two or more discharge chambers 36 in the direction of the central axis of the circulating ion beam are aligned.

The positioning guide 34 has a shape of a trapezoidal cross section as illustrated in FIG. 5, so that the position and rotation of the ion source 3 can be fixed. Note that instead of using the positioning guide 34, it is also possible to separately perform positioning by fixing with a flange or the like upstream in the insertion direction.

The cathode 33 is a material that easily emits electrons, and examples thereof include tungsten (W) and lanthanum hexaboride (LaB₆), but are not particularly limited thereto. In addition, the cathode 33 may be a cold cathode that is not overheated or a cathode that is forcibly heated by applying a current. However, the cold cathode is excellent in the lifetime of the cathode 33.

The discharge vessel 39 is preferably made of a material resistant to heat, such as tantalum (Ta) or copper (Cu) having excellent thermal conduction, but is not particularly limited thereto.

The first ion source 31 and the second ion source 32 operate as follows, and generate plasma to be the source of the ion beam.

A voltage is fed by the discharge power supply 21 between the cathode 33 and the container forming the discharge chamber 36. Electrons are emitted from the cathode 33 by the high voltage and are introduced into the discharge chamber 36 as a result toward the discharge vessel 39 having a positive potential as viewed from the cathode 33. Plasma is generated by collision between the sample gas 22 introduced through the gas pipe 24 and the electrons. At this time, due to the magnetic field B0 generated by the magnetic pole 10, the electrons perform a spiral motion, so that a stay time of the electrons in the discharge chamber 36 is extended, and plasma generation efficiency is further increased.

Next, a procedure of switching between the first ion source 31 and the second ion source 32 of the ion source 3 will be described.

First, the first ion source 31 is moved to the position of the radiofrequency acceleration electrode 8. In addition, a voltage is fed to the cathode 33 by the discharge power supply 21, the sample gas 22 is supplied to the discharge chamber 36 of the first ion source 31 by the gas switcher 23 to generate plasma, and the extraction beam 15 is extracted.

In a case where the extraction beam 15 is unstable or decreases, the ion source 3 is further inserted vertically to the main magnetic pole 1, and the second ion source 32 is moved to the position of the radiofrequency acceleration electrode 8 as illustrated in FIG. 6. At this time, since the ion source 3 is only moved in the direction parallel to the central axis, the vacuum state of the accelerator 50 may be maintained, and the time required for the exchange can be minimized.

Thereafter, the sample gas 22 is supplied to the discharge chamber 36 of the second ion source 32 by the gas switcher 23 to generate plasma, and the extraction beam 15 is extracted. At this time, since the sample gas 22 is not supplied to the discharge chamber 36 of the first ion source 31, ions are not generated.

In the present embodiment, the ion source 3 is inserted so as to penetrate the main magnetic pole 1 and the magnetic pole 10 provided with a hole. The plurality of ion sources (the first ion source 31 and the second ion source 32) can be installed while avoiding interference with the orbit 12 by being inserted from the upper portion of the main magnetic pole 1 and being installed to overlap in the insertion direction.

Here, the hole is provided in the main magnetic pole 1 and the magnetic pole 10, so that the distribution of the magnetic field B0 generated between the magnetic poles 10 is affected and changed. As the diameter of the hole is smaller, the influence on the magnetic field B0 is smaller, and correction can be easily performed by installing the shape of the magnetic pole 10, a separate iron piece, or the like. For this reason, it is necessary to reduce the hole formed in the magnetic pole 10, that is, it is necessary to reduce the outer diameter of the ion source 3.

Further, the circulating radius of the orbit 12 is determined by the strength of the magnetic field B0 generated between the magnetic poles 10. As the magnetic field strength increases, the circulating radius decreases. For example, in the case of proton ions accelerated at the magnetic field B0 of 2 Tesla and an acceleration voltage of 10 kilovolts, the circulating radius is 7.2 mm, and in the case of the magnetic field B0 of 6 Tesla, the circulating radius is 2.4 mm.

The outer diameter of the ion source 3 needs to be smaller than the circulating diameter (radius) in order to avoid a decrease in the current value due to collision of the circulating ion beam with the ion source 3 itself and to increase the utilization efficiency of the ion beam.

From the above, the cathode supporting tool 35 and the gas pipe 24 for supplying a voltage to the ion source 3 need to be installed in the narrow region. Therefore, a structure is formed such that a plurality of ion sources can be installed in the narrow region by supporting the cathode 33 by the common cathode supporting tool 35, supplying the sample gas 22 by the gas switcher 23 to generate plasma, and switching the ion source to be operated.

Next, a method of determining the timing of switching from the first ion source 31 to the second ion source 32 as illustrated in FIG. 6 or the timing of exchanging the ion source 3 itself will be described.

First, as a premise, a current monitor 17 that detects the current of the ion beam circulating in the orbit 12 circulating between the magnetic poles 10 is further provided. In addition, the discharge chamber 36 to be operated is switched according to the fluctuation of the current value detected by the current monitor 17.

More specifically, in a case where the current value detected by the current monitor 17 falls below a certain set value, the discharge power supply 21 is usually controlled to increase the voltage to be fed to the cathode 33. However, in a case where the beam current does not increase even if the voltage to be fed to the cathode 33 is increased, it can be determined that the first ion source 31 in use has reached its end of lifetime. Therefore, by moving the first ion source 31 and controlling the insertion mechanism, which is separately provided to use the second ion source 32 not currently operating, and the gas switcher 23, it is possible to supply the ion beam without stopping the ion source for a long time.

Furthermore, in a case where the extraction beam 15 is unstable or lowered even if the second ion source 32 is used, the ion source 3 itself needs to be exchanged. In this regard, when the ion source 3 itself is exchanged, the operation of the accelerator 50 is stopped, the atmosphere is opened, and the replacement is performed.

The monitoring of the current value detected by the current monitor 17 and the determination of the switching timing may be performed automatically on the apparatus side by the control apparatus (not illustrated for convenience of illustration), or may be performed by an operator by displaying a detection result on a display apparatus, and is not particularly limited.

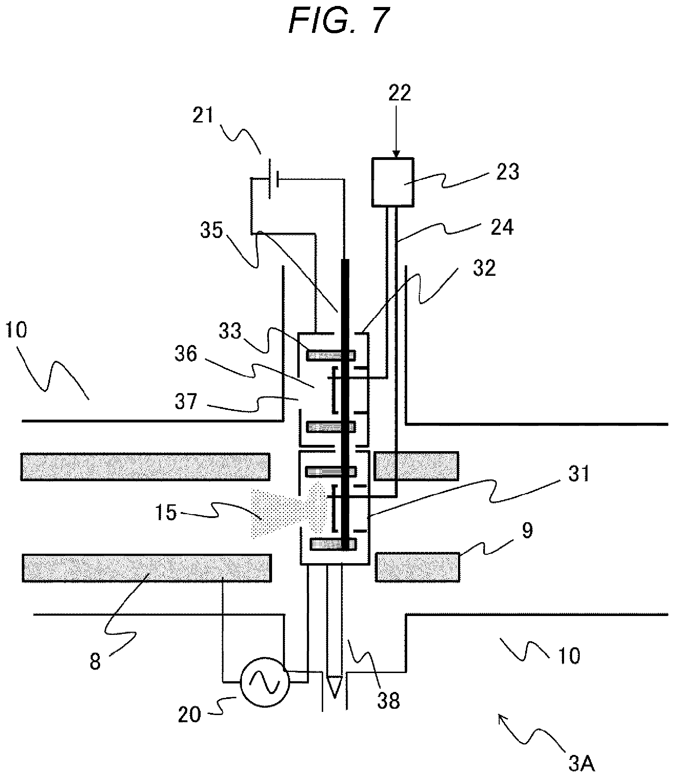

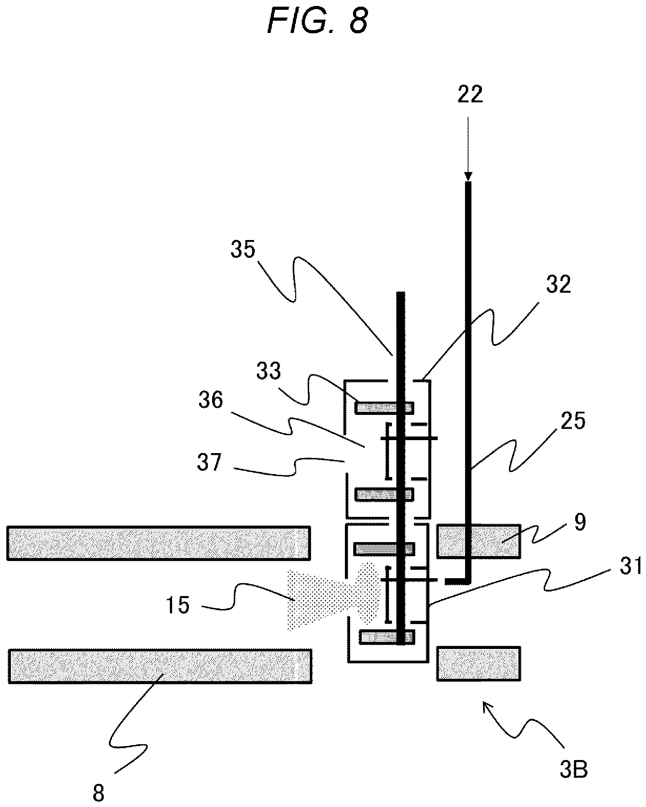

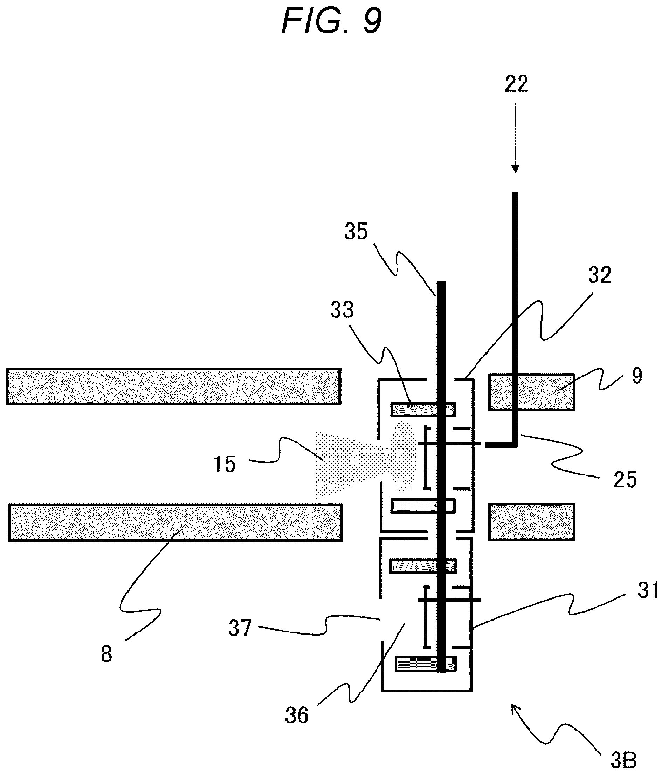

Next, a modification of the ion source 3 will be described with reference to FIGS. 7 to 9. FIG. 7 is a diagram schematically illustrating the case of being moved by the positioning pin, and FIGS. 8 and 9 are views schematically illustrating a case where the gas introduction pipe is integrated.

In an ion source 3A illustrated in FIG. 7, a positioning pin 38 is provided in the discharge chamber 36 arranged at the most end among at least two discharge chambers 36, and positioning including rotation is performed using the positioning pin 38.

The positioning pin 38 has a circular or semicircular cross section and is provided below the first ion source 31. The positioning pin 38 is passed through a reference hole provided in the magnetic pole 10 to position the ion source 3A. In addition, the ion source 3A is rotated about the positioning pin 38, so that the positioning pin can be used for position adjustment of the ion beam extraction direction of the ion source 3A, or the like. Note that a positioning pin may be provided on a surface of the magnetic pole 10 opposing the first ion source 31, and a reference hole may be provided on the side of the ion source 3.

The positioning pin 38 is desirably installed at a position corresponding to the rotation center of the ion source 3A, and is, for example, a lower portion of the extraction hole 37, the center of the ion source 3A, the center of the orbit 12, or the like.

In an ion source 3B illustrated in FIGS. 8 and 9, a gas pipe 25 for supplying a sample gas to the discharge chamber 36 is common to at least two discharge chambers 36.

FIG. 8 is an example of a case where the sample gas 22 is supplied to the first ion source 31 to generate plasma, and FIG. 9 is an example of a case where the ion source 3B is moved downward and the sample gas 22 is introduced into the second ion source 32 to generate plasma.

In the ion source 3B, the discharge chamber 36 moves in a direction parallel to the central axis as in FIGS. 4 to 6, and the gas pipe 25 connected from the discharge chamber 36 of the first ion source 31 and the discharge chamber 36 of the second ion source 32 to the surface of the discharge vessel 39 is provided. The present invention is not limited to the case where the gas pipe 25 is provided, and holes for supplying the sample gas may be common.

The gas pipe 25 is fixed to the ground electrode 9 so as to coincide with the positions of the gas supply holes provided in the first ion source 31 and the second ion source 32 when the first ion source 31 or the second ion source 32 moves to a height that coincides with the height of the radiofrequency acceleration electrode 8.

In a case where the first ion source 31 is used, as illustrated in FIG. 8, the first ion source is moved to a position where the gas introduction hole of the first ion source 31 and the gas pipe 25 can be connected to each other, and the sample gas 22 is introduced into the discharge chamber 36. In a case where the second ion source 32 is used, as illustrated in FIG. 9, the ion source 3B is moved downward and moved to a position where the gas introduction hole of the second ion source 32 coincides with the gas pipe 25. The sample gas 22 is introduced into the discharge chamber 36 of the second ion source 32 to generate plasma.

Even in a case where a plurality of ion sources are installed, the number of gas pipes 25 is only one, which is effective for installation in a narrow place.

Note **that,** in FIGS. 4 to 9, a case where the ion source 3, 3A, 3B is inserted from the upper portion of the main magnetic pole 1 has been described, but the ion source 3, 3A, 3B may be inserted from the lower portion toward the upper portion side of the main magnetic pole 1, and the same operation can be performed.

Next, effects of the present embodiment will be described.

The accelerator 50 of the present embodiment described above includes the opposing magnetic poles 10, the radiofrequency acceleration electrode 8, and the ion source 3, 3A, 3B installed between the opposing magnetic poles 10 and in which at least two discharge chambers 36 for linearly generating ions in parallel with the central axis of the circulating ion beam are arranged, and the discharge chamber 36 for extracting ions can be selected from among the at least two discharge chambers 36 by moving the ion source 3, 3A, 3B in a direction parallel to the central axis.

With such a configuration, in the cyclotron type accelerator 50 including the ion source 3, 3A, 3B inside the main magnetic pole 1, a plurality of ion sources can be installed in the ion source 3, 3A, 3B at the same position with respect to the positions of the radiofrequency acceleration electrode 8 and the magnetic pole 10, and even in a case where the ion sources are exchanged (the first ion source 31 to the second ion source 32), the position adjustment can be completed with only once, and without interference of the ion beam and without releasing to the atmosphere, the ion source can be changed sequentially to extract the ion beam. Therefore, since the number of ion sources can be increased as compared with the conventional internal ion source as described in PTL 1, it is possible to provide an accelerator capable of reducing the exchange frequency of the ion source as compared with the conventional internal ion source and significantly prolonging the lifetime of the internal ion source.

In addition, since the ion source 3, 3A, 3B can be integrally assembled and installed in series, the position can be adjusted at the same time by one installation, the maintainability is improved, the maintenance time and the maintenance cycle are shortened, and the effect of improving the operation rate of the apparatus can be obtained.

In addition, since there is no interference between the ion sources even under a high magnetic field or even when there are a plurality of radiofrequency acceleration electrodes, the loss of the current extracted from the accelerator is reduced, the extraction efficiency is improved, and the present invention can be applied to any circular accelerator.

Furthermore, in the technique as in PTL 1 described above, it is necessary to change the phase of the control parameter by 180° when the ion source is switched, but in the present invention, since the positional relationship of the ion beam in the circumferential direction does not change before and after the switching, there is also an advantage that the change of the phase is unnecessary and the control is easy.

In addition, the cathode 33 of the ion source 3, 3A, 3B is supported by the common cathode supporting tool 35 in at least two discharge chambers 36, and the sample gas is supplied only to the discharge chamber 36 to be operated in the ion source 3, 3A, 3B, so that the ion source 3, 3A, 3B can be configured to have a more space-saving configuration.

Furthermore, the gas pipe 25 for supplying the sample gas to the discharge chamber 36 is common to at least two discharge chambers 36, and the discharge chamber 36 is in a direction parallel to the central axis, whereby the ion source 3B can be configured to have a more space-saving configuration.

In addition, the current monitor 17 that detects the current of the circulating ion beam is further provided, and the discharge chamber 36 to be operated is switched according to the fluctuation of the current value detected by the current monitor 17, so that the first ion source 31 and the second ion source 32 can be switched at timing close to the lifetime as much as possible.

Furthermore, the ion source 3 includes the positioning guide 34 that aligns the positions in the direction of the central axis of the at least two discharge chambers 36, or the positioning pin 38 is provided in the discharge chamber 36 arranged at the most end among the at least two discharge chambers 36, so that the positioning of the ion source 3 in the direction of the central axis of the ion beam becomes easy, and the labor required for maintenance can be further saved.

In addition, since the extraction holes 37 for extracting ions from the discharge chamber 36 are linearly arranged side by side in parallel with the central axis, it is possible to reduce the adjustment when switching is performed from the first ion source 31 to the second ion source 32.

### <Others>

Note that the present invention is not limited to the above-described embodiments, and various modifications are included. For example, the above-described embodiments have been described in detail for easy understanding of the invention and are not necessarily limited to those having all the described configurations. In addition, a part of the configuration of a certain embodiment can be replaced with the configuration of another embodiment, and the configuration of another embodiment can be added to the configuration of a certain embodiment. It is possible to add, delete, and replace other configurations for a part of the configuration of each embodiment.

For example, the type of the accelerator 50 is not limited to a cyclotron type accelerator having a magnetic pole provided with unevenness on the magnetic pole 10, and the present invention can also be used for a synchrocyclotron type accelerator having a magnetic pole inclined from the center of the magnetic pole 10 and modulating the frequency of radio frequency acceleration.

The synchrocyclotron type accelerator is a type of accelerator in which a cyclotron is improved, and applies a frequency-modulated radiofrequency electric field to charged particles that circularly move between large magnetic poles to repeatedly accelerate the charged particles. In addition, at the speed of light, the mass of the accelerated particles increases due to a relativistic effect, and the period of circular motion of the charged particles in the magnetic field increases in proportion to the mass. The shift of the period from the radio frequency voltage caused by this is removed by modulating the frequency.

Since the synchrocyclotron type accelerator has the same configuration as the cyclotron type accelerator, the ion source 3 can be arranged in the main magnetic pole 1, and the same effect can be obtained.

### Reference Signs List

- 1: main magnetic pole
- 2: circular coil
- 3, 3A, 3B: ion source
- 6: vacuum chamber
- 7: acceleration gap
- 8: radiofrequency acceleration electrode
- 9: ground electrode
- 9A: notch
- 10: magnetic pole
- 12: orbit
- 15: extraction beam
- 17: current monitor
- 20: radiofrequency power supply
- 21: discharge power supply
- 22: sample gas
- 23: gas switcher
- 24, 25: gas pipe
- 31: first ion source
- 32: second ion source
- 33: cathode
- 34: positioning guide
- 35: cathode supporting tool
- 36: discharge chamber
- 37: extraction hole
- 38: positioning pin
- 39: discharge vessel
- 40: treatment table
- 45: patient
- 50: accelerator
- 52: beam transport system
- 54: irradiation apparatus
- 56: control apparatus
- 100: particle therapy system

## Claims

1. A circular accelerator (50) comprising:
opposing magnetic poles (10);
an acceleration electrode (8); wherein
the circular accelerator (50) is **characterized by** further comprising:
an ion source (3, 3A, 3B) which is arranged between the opposing magnetic poles (10) and in which at least two discharge chambers (36) are arranged side by side in a straight line parallel to a central axis of a circulating ion beam, wherein
the discharge chamber from which the ions are extracted is able to be selected from the at least two discharge chambers (36) by moving the ion source (3, 3A, 3B) in a direction parallel to the central axis.

2. The circular accelerator (50) according to claim 1, wherein
a cathode (33) of the ion source (3, 3A, 3B) is supported by a common support in the at least two discharge chambers (36), and
a sample gas (22) is supplied only to the discharge chamber to be operated in the ion source (3, 3A, 3B).

3. The circular accelerator (50) according to claim 1 or 2, wherein
a gas pipe (24, 25) for supplying the sample gas (22) to the discharge chamber is common to the at least two discharge chambers (36), and
the discharge chamber moves in the direction parallel to the central axis.

4. The circular accelerator (50) according to any one of claims 1 to 3, further comprising a current monitor (17) which detects a current of the ion beam, wherein
the discharge chamber to be operated is switched according to a fluctuation of a current value detected by the current monitor (17).

5. The circular accelerator (50) according to any one of claims 1 to 4, wherein
the ion source (3, 3A, 3B) includes a guide (34) for aligning positions of the at least two discharge chambers (36) in a direction of the central axis.

6. The circular accelerator (50) according to any one of claims 1 to 5, wherein
a positioning pin (38) is provided in the discharge chamber arranged at a most end among the at least two discharge chambers (36).

7. The circular accelerator (50) according to any one of claims 1 to 6, wherein
extraction holes (37) for extracting the ions from the discharge chamber are linearly arranged side by side in parallel with the central axis.

8. A particle therapy system comprising:
the circular accelerator (50) according to any one of claims 1 to 7;
a beam transport system (52);
an irradiation apparatus (54); and
a treatment table (40).

## Patentansprüche

1. Ringbeschleuniger (50), der umfasst:
gegenüberliegende Magnetpole (10);
eine Beschleunigungselektrode (8); wobei
der Ringbeschleuniger (50) **dadurch gekennzeichnet ist, dass** er ferner Folgendes umfasst:
eine Ionenquelle (3, 3A, 3B), die zwischen den gegenüberliegenden Magnetpolen (10) angeordnet ist und in der nebeneinander auf einer Geraden parallel zu einer Mittelachse eines zirkulierenden Ionenstrahls wenigstens zwei Entladungskammern (36) angeordnet sind, wobei
diejenige Entladungskammer, von der die Ionen extrahiert werden, aus den wenigstens zwei Entladungskammern (36) durch Bewegen der Ionenquelle (3, 3A, 3B) in einer Richtung parallel zu der Mittelachse ausgewählt werden kann.

2. Ringbeschleuniger (50) nach Anspruch 1, wobei
eine Katode (33) der Ionenquelle (3, 3A, 3B) durch einen gemeinsamen Träger in den wenigstens zwei Entladungskammern (36) getragen ist, und
nur in diejenige Entladungskammer, die in der Ionenquelle (3, 3A, 3B) betrieben werden soll, ein Messgas (22) zugeführt wird.

3. Ringbeschleuniger (50) nach Anspruch 1 oder 2, wobei
ein Gasrohr (24, 25) zum Zuführen des Messgases (22) zu der Entladungskammer für die wenigstens zwei Entladungskammern (36) gemeinsam ist, und
sich die Entladungskammer in der Richtung parallel zu der Mittelachse bewegt.

4. Ringbeschleuniger (50) nach einem der Ansprüche 1 bis 3, der ferner eine Stromüberwachungseinrichtung (17), die einen Strom des Ionenstrahls detektiert, umfasst, wobei
diejenige Entladungskammer, die betrieben werden soll, in Übereinstimmung mit einer Schwankung eines durch die Stromüberwachungseinrichtung (17) detektierten Stromwerts geschaltet wird.

5. Ringbeschleuniger (50) nach einem der Ansprüche 1 bis 4, wobei
die Ionenquelle (3, 3A, 3B) eine Führung (34) zum Ausrichten der Positionen der wenigstens zwei Entladungskammern (36) auf eine Richtung der Mittelachse enthält.

6. Ringbeschleuniger (50) nach einem der Ansprüche 1 bis 5, wobei
in der Entladungskammer ein Positionsstift (38) vorgesehen ist, der ganz am Ende unter den wenigstens zwei Entladungskammern (36) angeordnet ist.

7. Ringbeschleuniger (50) nach einem der Ansprüche 1 bis 6, wobei
nebeneinander parallel zu der Mittelachse Extraktionslöcher (37) zum Extrahieren der Ionen aus der Entladungskammer linear angeordnet sind.

8. Partikeltherapiesystem, das umfasst:
einen Ringbeschleuniger (50) nach einem der Ansprüche 1 bis 7;
ein Strahltransportsystem (52);
eine Bestrahlungsvorrichtung (54); und
einen Behandlungstisch (40).

## Revendications

1. Accélérateur circulaire (50) comprenant :
des pôles magnétiques opposés (10);
une électrode d'accélération (8) ; dans lequel
l'accélérateur circulaire (50) est **caractérisé en ce qu'**il comprend en outre :
une source d'ions (3, 3A, 3B) qui est agencée entre les pôles magnétiques opposés (10) et dans laquelle au moins deux chambres de décharge (36) sont agencées côte à côte dans une ligne droite parallèle à un axe central d'un faisceau d'ions en circulation, dans lequel
la chambre de décharge hors de laquelle les ions sont extraits est en mesure d'être sélectionnée à partir desdites au moins deux chambres de décharge (36) en déplaçant la source d'ions (3, 3A, 3B) dans une direction parallèle à l'axe central.

2. Accélérateur circulaire (50) selon la revendication 1, dans lequel
une cathode (33) de la source d'ions (3, 3A, 3B) est supportée par un support commun dans au moins deux chambres de décharge (36), et
un gaz échantillon (22) est alimenté uniquement à la chambre de décharge à actionner dans la source d'ions (3, 3A, 3B).

3. Accélérateur circulaire (50) selon la revendication 1 ou 2, dans lequel
un tube à gaz (24, 25) destiné à alimenter le gaz échantillon (22) à la chambre de décharge est commun auxdites au moins deux chambres de décharge (36), et
la chambre de décharge se déplace dans la direction parallèle à l'axe central.

4. Accélérateur circulaire (50) selon l'une quelconque des revendications 1 à 3, comprenant en outre un dispositif de surveillance de courant (17) qui détecte un courant du faisceau d'ions, dans lequel
la chambre de décharge à actionner est commutée en fonction d'une fluctuation d'une valeur de courant détectée par le dispositif de surveillance de courant (17).

5. Accélérateur circulaire (50) selon l'une quelconque des revendications 1 à 4, dans lequel
la source d'ions (3, 3A, 3B) inclut un guide (34) destiné à aligner des positions desdites au moins deux chambres de décharge (36) dans une direction de l'axe central.

6. Accélérateur circulaire (50) selon l'une quelconque des revendications 1 à 5, dans lequel
une broche de positionnement (38) est prévue dans la chambre de décharge agencée tout à l'extrémité desdites au moins deux chambres de décharge (36).

7. Accélérateur circulaire (50) selon l'une quelconque des revendications 1 à 6, dans lequel
des trous d'extraction (37) destinés à extraire les ions hors de la chambre de décharge sont agencés de manière linéaire côte à côte parallèlement à l'axe central.

8. Système de thérapie particulaire comprenant :
l'accélérateur circulaire (50) selon l'une quelconque des revendications1 à 7 ;
un système de transport de faisceau (52) ;
un appareil d'irradiation (54) ; et
une table de traitement (40).
